Europäisches Patentamt

European Patent Office

Office européen des brevets

(19)

(11) Numéro de publication: **0 255 426**
**A1**

## (12) DEMANDE DE BREVET EUROPEEN

(21) Numéro de dépôt: **87401681.9**

(22) Date de dépôt: **17.07.87**

(51) Int. Cl.⁴: **A 61 N 5/06**

(30) Priorité: **18.07.86 FR 8610470**

(43) Date de publication de la demande:
**03.02.88 Bulletin 88/05**

(84) Etats contractants désignés:
**DE ES FR GB IT NL SE**

(71) Demandeur: **JETSUN INTERNATIONAL S.á.r.l.**
**Avenue de Scandinavie Z.A. de Courtaboeuf**
**F-91994 Les Ulis Cédex (FR)**

**Routier, Jean-Denis**
**24, rue Saint-Bernard**
**F-75011 Paris (FR)**

(72) Inventeur: **Routier, Jean-Denis**
**24, rue Saint-Bernard**
**F-75011 Paris (FR)**

(74) Mandataire: **Schrimpf, Robert et al**
**Cabinet Regimbeau 26, Avenue Kléber**
**F-75116 Paris (FR)**

(54) **Dispositif émetteur de rayonnement ultraviolet et installation de traitement comprenant de tels dispositifs.**

(57) La présente invention concerne un dispositif émetteur de rayonnement ultraviolet, du type comprenant une source allongée (30) de rayonnement ultraviolet et un réflecteur (40) pour renvoyer les rayons émis vers lui par la source dans une direction générale d'émission donnée (Ox). Selon l'invention le réflecteur comporte un ensemble de lames réfléchissantes (401-406, 401'-406'), disposées en succession parallèlement à la source et en arrière de celle-ci sur une étendue angulaire d'environ 180°, toutes les lames réfléchissantes ayant même hauteur projetée (h) suivant la direction d'émission sur un plan perpendiculaire à celle-ci et une même étendue angulaire ( α ) autour de la source dans un plan perpendiculaire auxdites lames et parallèle à ladite direction d'émission, les lames étant orientées de manière à donner aux rayons réfléchis une direction moyenne parallèle à la direction d'émission, et un réflecteur secondaire (50) formant occulteur en forme de demi-cylindre s'étendant sur environ 180° en avant de la source, parallèlement à celle-ci, pour renvoyer vers ledit réflecteur (40) les rayons émis par la source vers l'avant.

L'invention concerne également une installation du genre cabine équipée de tels dispositifs.

Application au bronzage et au traitement thérapeutique de maladies de la peau.

FIG.5

EP 0 255 426 A1

**Description**

DISPOSITIF EMETTEUR DE RAYONNEMENT ULTRAVIOLET ET INSTALLATION DE TRAITEMENT COMPRENANT
DE TELS DISPOSITIFS.

La présente invention concerne d'une façon générale le traitement par rayonnement ultraviolet, et concerne plus particulièrement un dispositif pour émettre un rayonnement ultraviolet ainsi qu'une installation de traitement par rayonnement ultraviolet, du genre cabine, à usage thérapeutique, par exemple pour le traitement de certaines maladies de la peau, ou bien pour certains soins esthétiques comme le bronzage.

On connait déjà dans la technique antérieure un certain nombre de cabines ou analogues pour le traitement par rayonnement ultraviolet . Elles comprennent généralement un ensemble de tubes formant sources de rayonnement, par exemple des lampes à vapeur de mercure basse pression (telles que les références TL09 et TL 10 fabriquées par PHILIPS), qui s'étendent verticalement tout autour de l'espace central d'utilisation de la cabine. Des réflecteurs à section horizontale parabolique sont le cas échéant prévus à l'arrière des tubes pour renvoyer vers le centre de la cabine le rayonnement émis par les tubes dans leur direction. Enfin des moyens de filtrage peuvent être également prévus entre les tubes et l'espace central de la cabine, notamment afin d'éliminer certains rayonnements indésirables, comme les rayons infrarouges.

Un inconvénient majeur de ce type d'installation est inhérent à l'utilisation de tubes verticaux de grande longueur comme sources de rayonnement. On constate en effet que pour un tube orienté verticalement, l'intensité du rayonnement varie fortement suivant la hauteur à laquelle on se place par rapport à celui-ci. Elle est maximale au milieu du tube et minimale à ses extrémités, avec des différences de valeurs extrêment importantes.

En conséquence, lorsqu'un utilisateur debout dans la cabine est exposé au rayonnement, la région de son corps située au niveau du milieu des tubes, correspondant le plus souvent à l'abdomen, est exposée à un rayonnement bien supérieur à celui qui frappe ses jambes ou sa tête. Ceci est particulièrement indésirable pour l'application bronzage ou certaines applications thérapeutiques, dans lesquelles l'ensemble de la peau du sujet doit être exposée à un rayonnement relativement uniforme. Ainsi, soit le rayonnement est d'intensité suffisante à mi-hauteur mais insuffisante aux extrémités, soit au contraire un rayonnement suffisant en haut et en bas risque de créer à mi-hauteur, par un rayonnement d'intensité excessive, des brûlures sur la peau du patient.

Un autre inconvénient de ce genre de dispositif réside dans le fait que, le rayonnement étant essentiellement diffus, l'intensité reçue par le sujet dépend fortement de la distance par rapport aux sources de rayonnement. Les doses reçues sont alors mal maîtrisées, et les durées d'exposition ne peuvent être déterminées que très approximativement, avec des risques de surexpositions génératrices de brûlures.

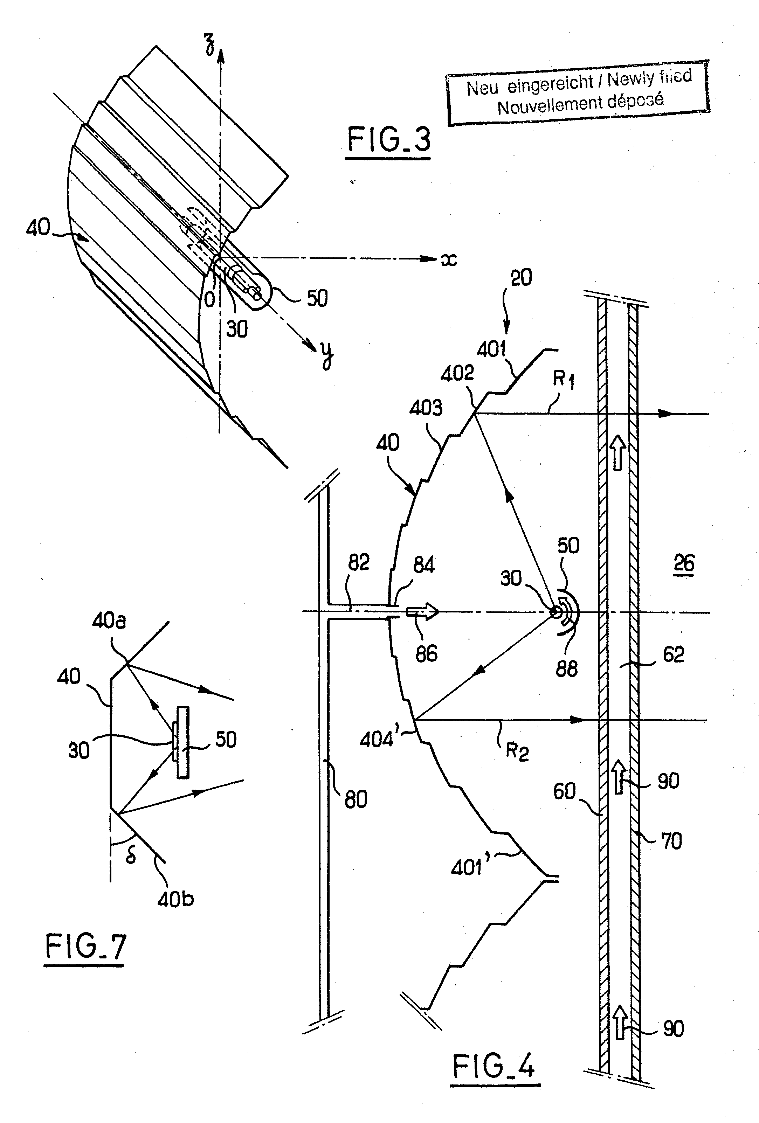

On connaît également dans la technique antérieure, par le brevet français n° 659 511 un dispositif destiné à produire un rayonnement à usage thérapeutique qui comprend un réflecteur en forme de paraboloïde, dont le foyer est situé sur la lampe. Pour corriger l'absence d'homogénéité du faisceau que ce type de réflecteur délivre, le réflecteur est divisé en un certain nombre de facettes circulaires d'inclinaisons déterminées. Mais il apparait que ce type de réflecteur ne délivre pas un faisceau tout à fait homogène. En particulier, la figure 3 montre que les angles couverts par les diverses facettes, ainsi que la largeur projetée de chacune des facettes, varient sensiblement du fond du réflecteur vers ses bords.

En outre, si le brevet spécifie les inclinaisons des facettes circulaires, il n'indique en revanche rien conconcernant la position de ces facettes par rapport à la source.

Un autre inconvénient de ce dispositif est le fait qu'environ la moitié du flux lumineux, émis par la lampe vers l'avant, diverge et est reçu directement par le patient. Il en résulte une intensité du rayonnement dans la direction d'émission bien plus importante au centre du réflecteur qu'à ses bords.

Enfin, le contour d'un tel réflecteur étant circulaire, il est impossible de juxtaposer une pluralité de ceux-ci pour obtenir un rayonnement homogène sur une grande surface.

La présente invention vise à pallier ces inconvénients et à proposer un dispositif émetteur de rayonnement ultraviolet et une installation de traitement équipée de tels dispositifs qui permettent d'obtenir une intensité lumineuse sensiblement constante quelle que soit la position d'une région exposée dans le sens de la hauteur, et plus généralement dans une direction perpendiculaire à la direction générale d'émission du rayonnement.

Un autre objet de la présente invention est de créer un rayonnement ultraviolet tel que la dose reçue par une région donnée du sujet soit sensiblement indépendante de la distance entre cette région et la ou les sources qui participent à son exposition.

A cet effet, la présente invention concerne tout d'abord un dispositif émetteur de rayonnement ultraviolet, du type comprenant une source allongée de rayonnement ultraviolet et un réflecteur pour renvoyer les rayons émis vers lui par la source dans une direction générale d'émission donnée (Ox), caractérisé en ce que le réflecteur comporte un ensemble de lames réfléchissantes, disposées en succession parallèlement à la source et en arrière de celle-ci sur une étendue angulaire d'environ 180°, toutes les lames réfléchissantes ayant même hauteur projetée (h) suivant la direction d'émission sur un plan perpendiculaire à celle-ci et une même étendue angulaire ($\alpha$) autour de la source dans un plan perpendiculaire auxdites lames et parallèle à ladite direction d'émission, les lames étant orientées de manière à donner aux rayons réfléchis une direction moyenne parallèle à la direction d'émission, et en ce qu'il comprend en outre un réflecteur secondaire formant occulteur en forme de demi-cylindre s'étendant sur environ 180° en avant de la source, parallèlement à celle-ci, pour renvoyer vers ledit réflecteur les rayons émis par la source vers l'avant.

L'invention concerne également une installation de traitement par rayonnement ultraviolet, caractérisée en ce qu'elle comprend une cabine dont au moins certaines parois sont définies par une superposition en colonne de dispositifs tels que définis ci-dessus.

L'invention concerne en outre un procédé de fabrication d'un réflecteur principal pour un dispositif du type exposé ci-dessus, caractérisé en ce qu'il comprend les étapes consistant à :
- extruder un profilé comportant un ensemble de facettes de support,
- fixer sur lesdites facettes des lames réfléchissantes et,
- découper le profilé et les lames à la longueur souhaitée.

L'invention sera mieux comprise à la lecture de la description détaillée suivante d'une forme de réalisation préférée de celle-ci, donnée à titre d'exemple et faite en référence aux dessins annexés, sur lesquels :
- la figure 1 est une vue de dessus schématique d'une installation de traitement aux rayons ultraviolets de la présente invention,
- la figure 2 est une vue en coupe verticale schématique selon la ligne II-II de la figure 1,
- la figure 3 est une vue en perspective d'un dispositif émetteur individuel de rayonnement ultraviolet de l'installation des figures 1 et 2,
- la figure 4 est une vue en coupe verticale détaillée d'une partie d'une colonne 18 équipée de dispositifs conformes à la figure 3,
- la figure 5 est une vue en coupe verticale détaillée illustrant la définition géométrique d'un élément formant réflecteur principal du dispositif émetteur des figures 3 et 4.
- la figure 6 est une vue en coupe verticale détaillée d'une variante de réalisation du réflecteur principal et,
- la figure 7 est une vue en coupe horizontale schématique d'une variante de réalisation du réflecteur de la figure 5 ou de la figure 6.
- la figure 8 est une vue en perspective illustrant une réalisation pratique du réflecteur de la figure 5 ou 6, et
- la figure 9 est une vue en élévation d'un élément de détail pouvant équiper l'installation de l'invention.

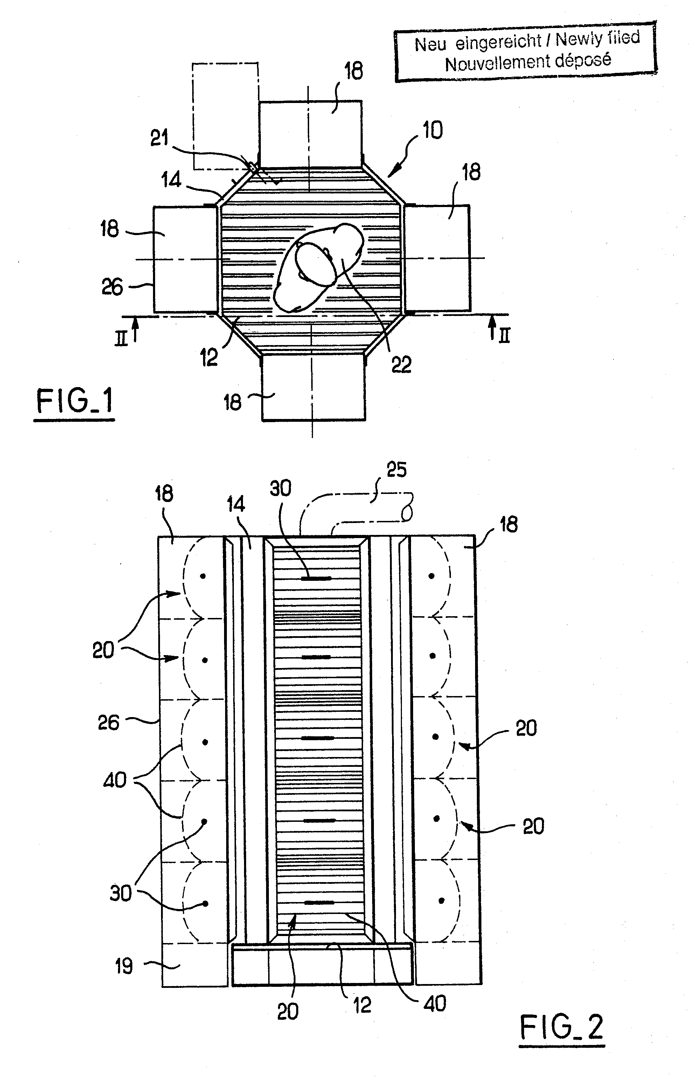

En référence aux dessins, et tout d'abord aux figures 1 et 2, une installation pour le traitement d'un sujet par rayonnement ultraviolet comprend une cabine 10 comportant un plancher 12, de forme octogonale dans le présent exemple, et des parois latérales 14. Quatre des parois latérales, opposées deux à deux, sont chacune définies par la face antérieure d'une colonne 18 constituée d'une superposition de dispositifs émetteurs de rayonnement ultraviolet selon la présente invention. Dans la présente forme de réalisation, chaque colonne 18 est consituée par cinq dispositifs émetteurs individuels superposés, désignés par la référence 20, portés par une embase 19.

L'une des colonnes 18 (en haut sur la figure 1) est montée sur charnières en 21 dans la région de l'un de ses bords latéraux verticaux, de telle sorte qu'elle fait office de porte d'accès à la cabine.

Un utilisateur ou sujet, indiqué en 22, debout au centre de la cabine, est ainsi soumis sur quatre côtés à un rayonnement ultraviolet, notamment à des fins thérapeutiques (par exemple pour le traitement du psoriasis) ou à des fins de bronzage ; dans les deux cas ci-dessus, la longueur d'onde du rayonnement sera par exemple de l'ordre de 330 à 350 nm (UVA). Il existe cependant d'autres cas de traitement thérapeutique où l'on devra utiliser un rayonnement ultraviolet "B".

Comme le montrent en particulier les figures 3 et 4, chaque dispositif émetteur 20 comprend une lampe 30, un réflecteur principal 40 et un réflecteur secondaire 50, formant également occulteur. La lampe 30 sera avantageusement du type brûleur à haute pression à vapeur de mercure et halogénure métallique, par exemple du type HPA, Héraeus ou BLV fabriquées par la société PHILIPS. Dans le présent exemple, il s'agit d'un tube de courte longueur orienté horizontalement et transversalement à la direction d'émission Ox.

Le réflecteur 40 est constitué par un ensemble de lames réfléchissantes, indiquées en 401, 402,.., 40n et 401', 402',..., 40n' qui s'étendent horizontalement parallèlement à l'axe de la lampe 30. Le réflecteur 40 s'étend angulairement sur environ 180° autour de la lampe, à l'arrière de celle-ci par rapport à l'espace d'utilisateur 26 (figure 4), et l'intervalle angulaire complémentaire, également de 180°, est occulté par le réflecteur secondaire 50, qui présente la forme d'un élément semi-cylindrique d'axe essentiellement confondu avec celui de la lampe et de rayon légèrement supérieur au rayon extérieur hors-tout de la lampe. Il est en outre pourvu à sa surface intérieure d'une couche réfléchissant les rayons ultraviolets. De la sorte, les rayons ultraviolets émis par la lampe vers l'avant sont réfléchis par le réflecteur secondaire 50 en direction du réflecteur principal 40, en passant à nouveau par la source ou au voisinage de celle-ci. On peut noter ici que la présence d'un tel occulteur 50 dans chaque dispositif élémentaire permet de diminuer la proportion de rayons infrarouges reçus par le sujet (ces rayons étant en partie absorbés par l'un et/ou l'autre des réflecteurs 40 et 50) et procure une protection physique du sujet en cas de bris de la lampe, notamment en l'absence des filtres éventuels envisagés ci-dessous.

Dans le présent exemple, on trouve en outre deux filtres 60 et 70 qui sont interposés sur le trajet des rayons ultraviolets tels que $R_1$ et $R_2$ réfléchis par le réflecteur 40 et dirigés vers l'espace 26.

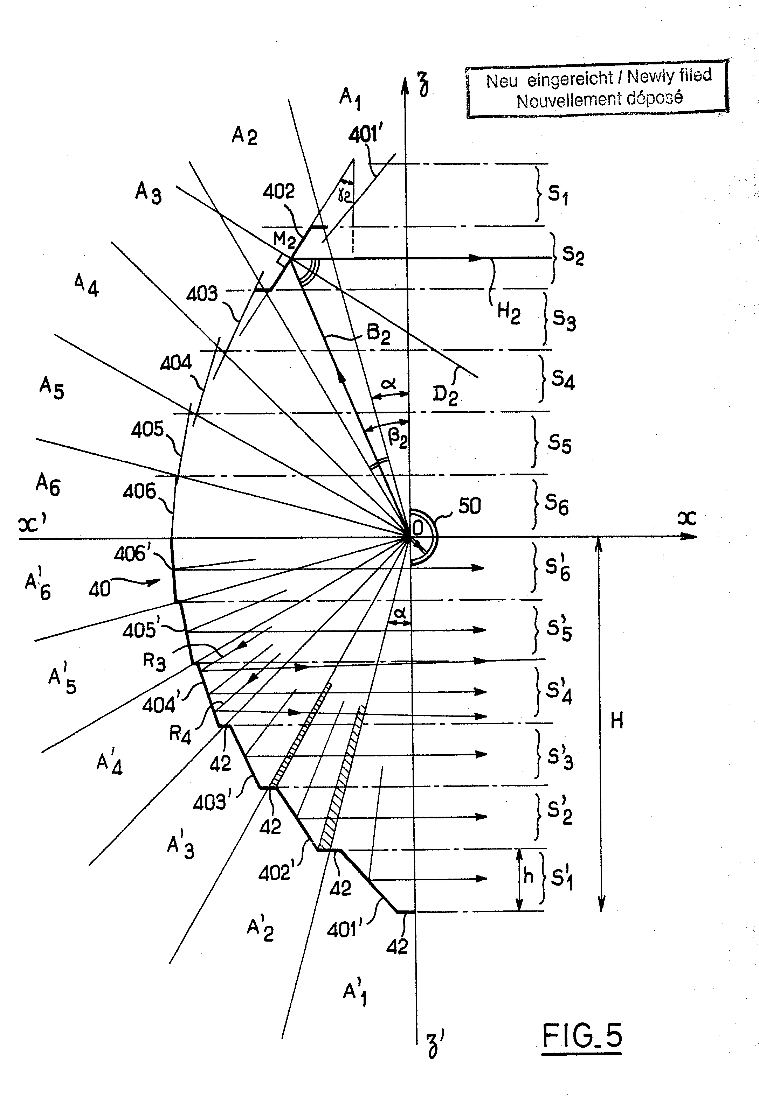

On va maintenant décrire en référence à la figure 5 la définition géométrique du réflecteur principal 40. Comme on peut l'observer sur cette figure, tous les rayons émanant de la source 30 (considérée comme ponctuelle en première approximation) vers l'arrière, directement ou après réflexion sur l'occulteur 50, sont interceptés par le réflecteur 40. L'espace angulaire correspondant de 180° est divisé en 2N secteurs de même ouverture angulaire. On a choisi dans le présent exemple N = 6, ce qui correspond à une ouverture angulaire $\alpha$ de 15° pour chaque secteur. Les douze secteurs angularies sont désignés par $A_1$ à $A_6$ et A  à A  respecti vement pour les moitiés supérieure et inférieure de l'espace angulaire de 180°. Est également prédéterminée la hauteur totale 2H du réflecteur 40, qui est quant à elle divisée horizontalement en 2N parties en forme de bandes de même hauteur $h = H/N = H/6$.

Chaque lame individuelle 401,..., 406 et 401',..., 406', qui dans le présent exemple est de profil droit et s'étend horizontalement et transversalement à la direction d'émission Ox (c'est-à-dire parallèlement à y'Oy), est définie géométriquement d'une part par la position de sa ligne médiane horizontale et d'autre part par son inclinaison par rapport à la verticale z'Oz.

La figure 5 illustre plus particulièrement la définition géométrique de la lame 402. En projection dans le plan xOz de cette figure, conformément à la présente invention, le milieu $M_2$ de la lame 402 est situé à l'intersection de la bissectrice, notée $B_2$, du secteur $A_2$ avec la ligne horizontale $H_2$ passant par le milieu de la bande de sortie correspondante $S_2$ en direction verticale. L'inclinaison de la lampe 402 par rapport à la verticale, désignée par l'angle $\gamma_2$, est telle que la surface de la lame est perpendiculaire à la bissectrice $D_2$ de l'angle formé par les droites $B_2$ et $H_2$, comme représenté. Dans le cas présent, on a $\beta_2 = 22{,}5°$, et l'on pourrait démontrer par un raisonnement géométrique élémentaire que l'on obtient pour $\gamma_z$ une valeur de 33,75°.

Les projections dans le plan xOz des lames successives peuvent ainsi être tracées en reproduisant la procédure ci-dessus, pour aboutir à la configuration telle que représentée.

D'une façon générale, pour un réflecteur divisé en 2N lames et ayant une hauteur globale 2H, on pourrait démontrer que, pour la lame 40n :
- les coordonnées dans le plan xOz tel que représenté du point médian $M_n$ de la lame considérée sont :
$X_n = -(H/2N)/(2N - 2n + 1) . tg(\pi.(2n - 1)/4N)$
$Z_n = (H/2N)/(2N - 2n + 1)$ ; et
- l'inclinaison $\delta_n$ de la lame considérée par rapport à la verticale z'Oz est :
$\gamma_n = (\pi/4).(1 - (2n - 1)/2N)$
n pouvant prendre toute valeur entière comprise entre 1 et N.

Il est bien entendu que le raisonnement et les calculs effectués ci-dessus pour la moitié supérieure du secteur angulaire de 180° s'appliquent également à la moitié inférieure, qui sera dans la pratique parfaitement symétrique de ladite moitié supérieure par rapport à l'axe d'émission x'Ox.

Comme le montre la figure 5, les limites horizontales supérieure et inférieure de chaque lame 40n de part et d'autre de sa ligne médiane sont déterminées de manière à ce que sa projection horizontale sur z'Oz soit confondue avec la bande de sortie $S_n$ associée. Les différences de profondeur qui existent à la jonction entre deux lames successives sont compensées par des parties horizontales du réflecteur, désignées par la référence 42, qui seront dans l'ensemble de faible profondeur et n'engendreront que de petites quantités de rayons parasites. Par ailleurs, la perte de rendement du dispositif du fait de la présence de ces parties 42 qui ne participent pas au rayonnement de sortie est relativement limitée. On a représenté à cet égard, en association avec le secteur angulaire $A'_2$ et la lame 402', les angles morts correspondant à ces parties de raccordement (zones hachurées).

Par ailleurs, dans le réflecteur défini ci-dessus, on peut observer que, si un rayon incident suivant la bissectrice $B_n$ est toujours réfléchi dans une direction rigoureusement horizontale, du fait de la définition même des lames du réflecteur, en revanche il n'en est pas de même pour d'autres rayons lumineux dirigés depuis la source vers la même lame. Ceci est dû au fait que chaque lame présente ici un profil droit. Ainsi, deux rayons incidents $R_3$ et $R_4$ et leurs rayons réfléchis par la lame 404' sont représentés sur la figure 5. On observe une légère divergence de ces rayons réfléchis.

Ainsi, conformément à la définition plus haut, et en négligeant les phénomènes secondaires décrits ci-dessus, chaque lame 40n est affectée géométriquement d'une part à un secteur angulaire $A_n$ prédéterminé et d'ouverture $\alpha$ constante (aux angles morts près) de la plage d'émission, non occultée de la lampe, et d'autre part à une bande $S_n$ de sortie de rayonnement de hauteur constante. On peut en déduire que, puisque l'énergie du rayonnement est bien entendu la même d'un secteur angulaire à l'autre, l'énergie lumineuse de sortie sera sensiblement la même d'une bande à l'autre (en négligeant pour le moment les effets de divergence des rayons réfléchis par les lames). Ainsi, l'intensité lumineuse reçue par une région donnée du sujet sera toujours la même quelle que soit la position de cette région en hauteur par rapport à la source. En outre, les rayons ultraviolets n'étant que faiblement divergents à la sortie du dispositif émetteur, la dose reçue sera également sensiblement constante quelle que soit la distance horizontale entre la région exposée et la source. En outre, la proportion de rayons soumis à une réflexion dioptrique sur les filtres sera extrêmement faible.

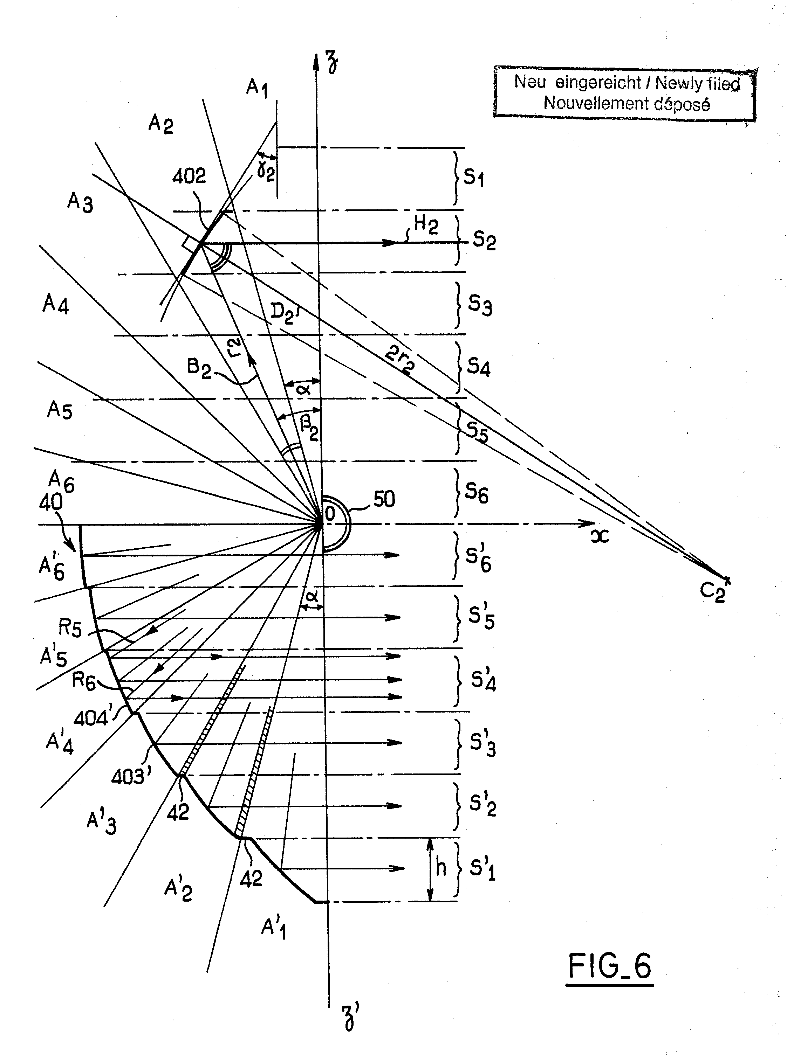

La figure 6 illustre une variante de réalisation de l'invention dans laquelle la légère divergence mentionnée plus haut est supprimée, ou tout au moins fortement atténuée. Selon cette variante, chaque lame, au lieu d'avoir un profil droit, présente un profil courbe permettant de rabattre vers la direction d'émission souhaitée Ox les rayons ultraviolets émis par la source qui sont écartés de la bissectrice $B_n$ considérée.

De façon préférée, ce profil courbe est constitué par un arc de cercle dont le centre $C_n$ est situé sur la bissectrice $B_n$ à une distance $2r_n$ du point M, $r_n$ étant le rayon moyen de la lame considérée vis-à-vis de la source (origine 0), c'est-à-dire la distance $OM_n$. Parallèlement à la représentation de la figure 5, on observe cette fois-ci que deux rayons $R_5$ et $R_6$ frappant la lame 404' respectivement de part et d'autre de la bissectrice associée sont réfléchis dans une direction pratiquement parallèle à l'axe Ox.

Bien entendu, une annulation totale de la divergence des lames pourrait être obtenue en donnant à celles-ci des profils paraboliques différents appropriés. L'homme de l'art sera à même, à la lumière des enseignements ci-dessus, de déterminer ces profiles individuellement pour chaque lame, en fonction de sa position et de son inclinaison moyenne.

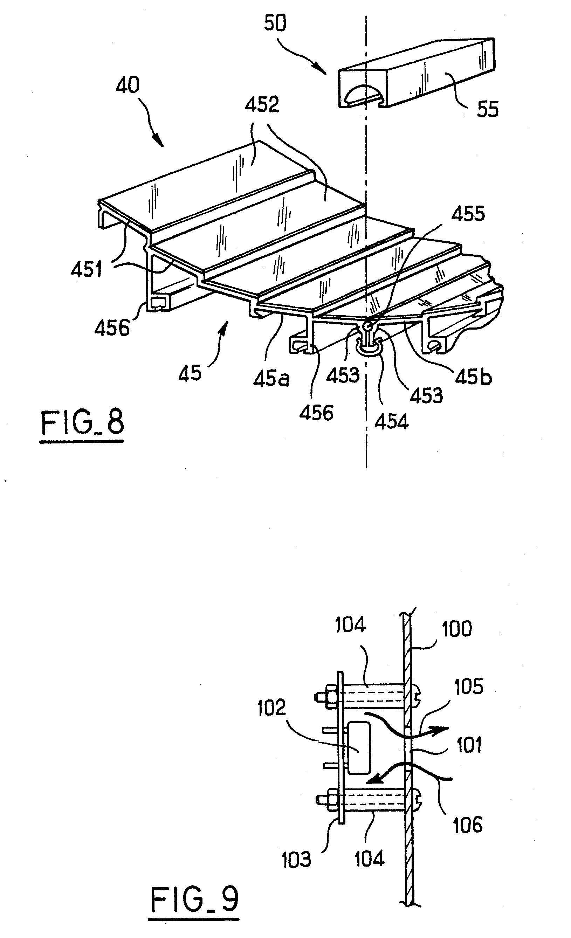

La figure 7 illustre, en vue en plan en coupe partielle, une variante de réalisation du réflecteur 40. Selon cette variante, afin d'atténuer la dispersion du flux de rayonnement ultra-violet en direction horizontale, les bords latéraux 40a, 40b du réflecteur sont rabattus d'un angle δ déterminé. Des essais ont démontré qu'un angle δ de 22° donnait les résultats optimaux.

De retour à la figure 4, l'installation de l'invention comprend comme on l'a indiqué plus haut deux filtres 60, 70 en forme de plaques parallèles séparées par un interstice 62 de largeur prédéterminée. Les filtres 60, 70 sont destinés en combinaison à éliminer certaines longueurs d'onde indésirables du rayonnement (infrarouges, ultraviolets B ou C, lumière visible). Bien connus de l'homme de la technique,ils ne seront pas décrits en détail.

On trouve en outre des moyens de refroidissement, qui sont rendus nécessaires par le fait que les lampes à haute pression à vapeur de mercure et halogénure métallique envisagées dans la présente invention fonctionnent à très haute température, en dégageant un rayonnement infrarouge intense. Ces moyens de refroidissement comprennent tout d'abord, pour chaque colonne 18, un conduit 80 qui est alimenté par des moyens appropriés (non représentés) en air froid sous une pression supérieure à la pression atmosphérique. Le conduit 80 s'étend verticalement dans la colonne, derrière les réflecteurs 40, et est relié par des embranchements 82 à des buses 84 prévues dans chaque réflecteur 40 dans la région du fond de celui-ci. Un jet d'air frais est ainsi engendré (flèche 86), qui est dirigé vers la lampe associée pour la refroidir. On peut noter à cet égard que le réflecteur secondaire 50, qui définit avec la lampe un canal semi-annulaire, favorise le refroidissement de la lampe en créant une circulation d'air secondaire suivant la flèche 88, cette circulation refroidissant la partie de la lampe qui n'est pas exposée au jet sortant de la ou des buses. Une telle circulation secondaire sera favorisée en décalant légèrement l'orientation des jets 86 vers le haut et vers le bas.

On prévoira de préférence deux buses 84 par réflecteur. De façon avantageuse, ces buses seront situées dans la région du milieu du réflecteur 40, c'est-à-dire dans la zone d'ombre vis-à-vis de l'extérieur due à la présence du réflecteur secondaire 50. Les défauts optiques qu'elles sont susceptibles d'engendrer seront ainsi imperceptibles.

L'air pourra être évacué en haut de chaque colonne 18 par un conduit commun 25 (voir figure 2).

Une autre solution (non représentée) pour effectuer ce refroidissement pourra consister, en conservant les buses 84, à mettre le compartiment de chaque colonne qui est situé derrière les réflecteurs 40, entre ceux-ci et la paroi arrière 26 de chaque colonne, en communication avec l'air ambiant à pression atmosphérique, et à créer dans le compartiment situé entre les réflecteurs 40 et les filtres une dépression, par exemple à l'aide d'un ventilateur situé dans un conduit d'extraction analogue au conduit 25 ou en aval de ce dernier. Un jet d'air frais sera de la même manière que décrit plus haut dirigé vers les lampes.

En référence de nouveau à la figure 4, on a représenté des flèches 90 qui symbolisent une circulation d'air frais entre les deux filtres 60, 70. Une telle circulation sera nécessaire en pratique lorsque l'un ou l'autre de ces filtres devra arrêter une quantité importante de rayons infrarouges et aura par conséquent une tendance à s'échauffer. Cette circulation s'effectuera par exemple en parallèle de la circulation d'air de refroidissement des lampes décrite plus haut.

Cependant, dans le cas où l'on utilisera des lampes émettant peu de rayonnement infrarouge et/ou des réflecteurs absorbant une grande partie de ce rayonnement, cette circulation d'air entre les filtres pourra être omise. Par ailleurs, dans le cas où les caractéristiques spectrales de l'émission de la lampe 30 et les caractéristiques d'absorption des réflecteurs 40, 50 le permettront, les filtres pourront être omis.

De façon avantageuse, on peut équiper l'installation conforme à l'invention de moyens de coupure de l'alimentation dans le cas où la température intérieure s'élève excessivement, notamment en cas de défaillance de l'un ou l'autre des moyens de ventilation décrits ci-dessus.

On a représenté sur la figure 9 un dispositif interrupteur thermique qui est très avantageusement utilisé avec les moyens de ventilation par dépression envisagés plus haut.

On a indiqué en 100 un élément de carrosserie de l'installation qui sépare l'intérieur d'une colonne de réflecteurs de l'atmosphère extérieure. Un orifice 101 de petit diamètre est formé dans la carrosserie et, en regard de celui-ci, un élément interrupteur thermique 102 de type connu en soi, est monté sur une plaquette de support 103 portée elle-même par des colonnettes 104 thermiquement isolantes.

De façon préférée, ce dispositif est monté dans la région supérieure d'une colonne de réflecteurs. Son comportement est le suivant : lorsque la ventilation par dépression fonctionne correctement, de l'air frais est aspiré de l'extérieur vers l'espace intérieur de ladite colonne (flèche 105) et l'interrupteur thermique 102, constamment balayé par le courant d'air frais, reste fermé et autorise ainsi l'alimentation des lampes des divers réflecteurs.

Inversement, lors d'une défaillance de la ventilation, par exemple son arrêt accidentel, l'air chaud qui s'accumule dans l'espace intérieur monte par convection et s'évacue à contre courant (flèche 106). Le thermo-rupteur 102 balayé par cet air chaud s'ouvre alors et interrompt l'alimentation des lampes en tenant lieu de dispositif de sécurité.

Bien entendu, on peut prévoir par exemple un dispositif thermo-rupteur par colonne, ou bien un dispositif unique pour l'ensemble de l'installation.

Le réflecteur 40 pourra par exemple être réalisé en aluminium de haute pureté, la couche réfléchissante étant obtenue par un traitement électrolytique assurant un pouvoir réfléchissant élevé et un poli spéculaire. Les rayonnements ultraviolets diffus seront ainsi avantageusement en très faible proportion, au profit des qualités mentionnées plus haut en matière de contrôle de la dosimétrie.

Bien entendu, dans le cas où les caractéristiques du rayonnement (notamment l'énergie du rayonnement infrarouge, liée à la puissance des lampes et à leur distance par rapport au réflecteur) le permettront, les réflecteurs pourront également être réalisés en matière plastique moulée par injection, la couche intérieure réfléchissante étant réalisée par métallisation sous vide.

On a représenté sur la figure 8 un réflecteur principal, ainsi que le réflecteur secondaire associé, destiné à être placé devant la lampe, réalisés par un procédé particulièrement avantageux constituant un autre aspect de la présente invention.

Le réflecteur 40 comprend un support 45 réalisé par extrusion, de préférence en un alliage d'aluminium, doté d'une excellente stabilité à la température.

Comme on peut l'observer, le support 45 comprend un ensemble de facettes planes 451 dont la géométrie correspond par exemple à celle qui est représentée sur la figure 5.

Sur chaque facette est fixée, de préférence par collage, une bande plane réfléchissante de largeur correspondante, respectivement 452.

L'ensemble décrit ci-dessus se présente sous la forme d'un profilé de grande longueur, que l'on coupe, par exemple par sciage, à la longueur souhaitée.

Les avantages offerts par un tel procédé de fabrication sont les suivants :
- comme l'exige la conception spécifique du réflecteur de la présente invention, fondée sur des développements géométriques de grande précision, il permet d'obtenir un excellent parallélisme des facettes sur toute leur longueur, ainsi qu'une excellente planéité. De façon associée, l'extrusion du support permet d'obtenir les angles de réflexion souhaités avec une très grande précision, de même que les positions dans l'espace des diverses lames,
- chaque réflecteur (ou chaque demi-réflecteur, comme on va le voir ci-dessous) se présente sous la forme d'une coquille unitaire, par conséquent d'une grande robustesse,
- le procédé est économique,
- l'adaptation à des cabines de dimensions diverses est rendue extrêmement aisée : il suffit de découper les éléments à la longueur particulière requise ; de façon associée, des coupes obliques peuvent être réalisées très facilement pour obtenir, dans la réalisation représentée schématiquement sur la figure 7, une jonction satisfaisante entre deux éléments réfléchissants adjacents.

Dans la réalisation illustrée sur la figure 8, chaque réflecteur est réalisé par l'assemblage central, de deux demi-coquilles 45a, 45b, à l'aide de pattes 453 maintenues assemblées par des clips élastiques 454 ou analogues et à l'aide de rainures 455 en vis-à-vis qui, en recevant un joint de calage approprié (non représenté), assurent un positionnement mutuel correct des deux demi-coquilles.

En outre, des pieds de soutien 456, prévus à intervalles réguliers à l'arrière des coquilles permettent d'assurer un positionnement et une fixation convenables de chaque réflecteur dans son caisson.

Le réflecteur secondaire 50 peut également être réalisé par extrusion d'un profilé 55 dont la forme intérieure est semi-cylindrique et qui peut recevoir, pour assurer la réflexion, soit un traitement réfléchissant approprié (par exemple métallisation sous vide), soit un ensemble de petites lames réfléchissantes planes. Les avantages indiqués plus haut quant au réflecteur principal sont sensiblement les mêmes.

La présente invention n'est nullement limitée aux formes de réalisation décrites ci-dessus et représentées sur les dessins, mais inclut dans son cadre toute variante ou modification venant à l'esprit de l'homme de l'art. En particulier, les paramètres indiqués plus haut en relation avec la définition du réflecteur 40, notamment le nombre de lames 2N et la dimension verticale 2H du réflecteur, pourront prendre toute valeur appropriée.

En outre, les expression "vertical", "horizontal", etc, utilisées dans la présente description sont à considérer dans un sens relatif, étant bien entendu que l'on pourra donner à chaque dispositif émetteur de rayonnement ultraviolet individuel toute orientation appropriée. Les seules limitations seront celles qui sont dues aux conditions de fonctionnement des lampes, dont certains types ne peuvent fonctionner qu'en orientation horizontale.

En outre l'installation de la présente invention pourra être conçue pour effectuer soit une exposition globale du sujet, toutes les lampes fonctionnant simultanément, soit une exposition limitée à des régions choisies, par des commutations indépendantes des lampes individuellement ou par groupes.

## Revendications

1. Dispositif émetteur de rayonnement ultraviolet, du type comprenant une source allongée (30) de rayonnement ultraviolet et un réflecteur (40) pour renvoyer les rayons émis vers lui par la source dans une direction générale d'émission donnée (Ox), caractérisé en ce que le réflecteur comporte un ensemble de lames réfléchissantes (401-406, 401'- 406'), disposées en succession parallèlement à la source (30) et en arrière de celle-ci sur une étendue angulaire d'environ 180°, toutes les lames réfléchissantes ayant même hauteur projetée (h) suivant la direction d'émission sur un plan perpendiculaire à celle-ci et une même étendue angulaire ($\alpha$) autour de la source dans un plan perpendiculaire auxdites lames et parallèle à ladite direction d'émission, les lames étant orientées de manière à donner aux rayons réfléchis une direction moyenne parallèle à la direction d'émission, et en ce qu'il comprend en outre un réflecteur secondaire (50) formant occulteur en forme de demi-cylindre s'étendant sur environ 180° en avant de la source (30), parallèlement à celle-ci, pour renvoyer vers ledit réflecteur (40) les rayons émis par la source vers l'avant.

2. Dispositif selon la revendication 1, caractérisé en ce que chaque lame (401-406, 401'-40 ') présente en section transversale un profil droit.

3. Dispositif selon la revendication 1, caractérisé en ce que chaque lame (401-406, 401'-406') présente en section transversale un profil courbe.

4. Dispositif selon la revendication 3, caractérisé en ce que ledit profil courbe est un arc de cercle d'un rayon approximativement égal au double de la distance entre la source (30) et la lame (401-406, 401-406') considérée.

5. Dispositif selon l'une quelconque des revendications 1 à 4, caractérisé en ce que l'orientation de chaque lame est au moins localement perpendiculaire à la bissectrice ($D_n$) de l'angle défini par la bissectrice ($B_n$) de ladite étendue angulaire ($A_n$) et par la direction d'émission ($H_n$).

6. Dispositif selon l'une quelconque des revendications 1 à 5, caractérisé en ce que les lames adjacentes (401-406, 401'-406') sont reliées entre elles par des parties de raccordement (42) parallèles à la direction d'émission (Ox), le réflecteur (40) étant réalisé d'un seul tenant.

7. Dispositif selon l'une quelconque des revendications 1 à 6, caractérisé en ce qu'il comprend en outre des moyens de filtrage (60, 70) interposés sur le trajet des rayons ultraviolets réfléchis par le réflecteur (40).

8. Dispositif selon la revendication 7, caractérisé en ce qu'il comprend au moins deux filtres parallèles (60, 70) séparés par un espace intersticiel, et des moyens de refroidissement des filtres par circulation d'air dans ledit espace.

9. Dispositif selon l'une quelconque des revendications précédentes, caractérisé en ce que la source (30) est un brûleur à haute pression à vapeur de mercure et halogénure métallique, et en ce qu'il comprend des moyens de renfroidissement du brûleur constitués par au moins une buse (84) formée dans le réflecteur (40).

10. Dispositif selon la revendication 9, caractérisé en ce que la ou les buses sont formées à la hauteur de la source (30).

11. Installation de traitement par rayonnement ultraviolet, caractérisée en ce qu'elle comprend une cabine (10) dont au moins certaines parois sont définies par une superposition en colonne (18) de dispositifs (20) selon l'une quelconque des revendications précédentes.

12. Installation de traitement selon la revendication 11, caractérisé en ce que la direction d'émission (Ox) est horizontale et en ce que les lames (401-406, 401'-406') s'étendent horizontalement transversalement à ladite direction d'émission.

13. Procédé de fabrication d'un réflecteur principal pour un dispositif selon l'une des revendications 1 à 10, caractérisé en ce qu'il comprend les étapes consistant à :
- extruder un profilé (45) comportant un ensemble de facettes (451) de support,
- fixer sur lesdites facettes des lames réfléchissantes (452), et
- découper le profilé et les lames à la longueur souhaitée.

14. Réflecteur principal obtenu par le procédé selon la revendication 13, caractérisé en ce que le profilé (45) est en alliage d'aluminium.

15. Réflecteur selon la revendication 14, caractérisé en ce qu'il comprend deux demi-profilés (45a, 45b) assemblés centralement.

16. Réflecteur selon l'une des revendications 14 et 15, caractérisé en ce que le profilé (45) comprend des pieds (456) de positionnement et de fixation.

17. Réflecteur selon l'une des revendications 14 à 16, caractérisé en ce que les facettes (451) et les lames réfléchissantes (452) sont planes.

0255426

FIG_1

FIG_2

0255426

FIG_3

FIG_7

FIG_4

0255426

FIG.5

0255426

Neu eingereicht / Newly filed
Nouvellement déposé

FIG_6

0255426

FIG_8

FIG_9

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | FR-A- 659 511 (HANOVIA) <br><br> * Page 1, lignes 19-33; page 3, lignes 15-36; figures 1,3 * <br><br> --- | 1,2,5, 6 | A 61 N 5/06 |
| A | DE-A-2 529 983 (KOCKOTT) <br> * Page 6, lignes 4-21; figure 1 * <br><br> --- | 1 | |
| A | US-A-4 499 529 (FIGUEROA) <br> * Colonne 2, lignes 21-62; fig-ures 1,2 * <br><br> --- | 1 | |
| A | DE-A-2 804 228 (WOLFF) <br><br> * Page 9, lignes 1-19; page 9, ligne 33 - page 10, ligne 6; page 11, lignes 13-15; figures 1,2,4,7 * <br><br> --- | 1,7,9, 11,12 | |
| A | DE-A-2 615 446 (ZIMMERMANN) <br> * Page 5, ligne 16 - page 6, ligne 2; figure 2 * <br><br> --- | 1-3 | DOMAINES TECHNIQUES RECHERCHES (Int. Cl.4) <br><br> A 61 N <br> A 61 V |
| A | WO-A-8 404 796 (SKOGLUND) <br> * Page 5, lignes 1-18; figures 1,4 * <br><br> --- | 1 | |
| A | DE-U-8 508 206 (WEINER) <br> * Page 6, ligne 10 - page 7, ligne 25; figures 1,2 * <br><br> --- -/- | 1,7-9 | |

Le présent rapport de recherche a été établi pour toutes les revendications

| Lieu de la recherche <br> LA HAYE | Date d'achèvement de la recherche <br> 20-10-1987 | Examinateur <br> SCHMIERER U.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03 82

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-2 352 747 (INFRARÖDTEKNIK AB) <br> * Page 3, ligne 25 - page 4, ligne 2; figure 1 * | 1,15 | |
| A | AT-A- 359 163 (ZUMTOBEL AG) <br><br> * Page 3, lignes 10-21; page 3, lignes 26-31; page 4, lignes 9-17; figures 1,2 * | 1,13, 14,16 | |
| A | ELEKTROTECHNIK, vol. 59, no. 12, 24 juin 1977, page 67; SEMPERLUX: "Spiegelprofil-System" * En entier * | 14 | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 20-10-1987 | SCHMIERER U.J. |